(19) Europäisches Patentamt
European Patent Office
Office européen des brevets

(11) Veröffentlichungsnummer: **0 005 756**
**B1**

(12) # EUROPÄISCHE PATENTSCHRIFT

(45) Veröffentlichungstag der Patentschrift: **14.10.81**

(51) Int. Cl.³: **C 07 D 317/46,** A 01 N 43/30

(21) Anmeldenummer: **79101465.7**

(22) Anmeldetag: **14.05.79**

(54) **5-Arylamino-2,2-difluor-benzodioxole, Verfahren zu ihrer Herstellung und ihre Verwendung zur Schädlingsbekämpfung.**

(30) Priorität: **26.05.78 DE 2823168**

(43) Veröffentlichungstag der Anmeldung: **12.12.79 Patentblatt 79/25**

(45) Bekanntmachung des Hinweises auf die Patenterteilung: **14.10.81 Patentblatt 81/41**

(84) Benannte Vertragsstaaten: **BE CH DE FR GB IT NL**

(56) Entgegenhaltungen:

**keine Entgegenhaltungen**

(73) Patentinhaber: **BAYER AG, Zentralbereich Patente, Marken und Lizenzen, D-5090 Leverkusen 1, Bayerwerk (DE)**

(72) Erfinder: **Hartmann, Alfons, Dr., Poststrasse 35, D-6645 Beckingen 4 (DE)**
Erfinder: **Marhold, Albrecht, Dr., Carl-Duisberg-Strasse 329, D-5090 Leverkusen 1 (DE)**
Erfinder: **Lantzsch, Reinhard, Dr., Heymannstrasse 32, D-5090 Leverkusen 1 (DE)**
Erfinder: **Hammann, Ingeborg, Dr., Belfortstrasse 9, D-5000 Köln 1 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

EP 0 005 756 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

5-Arylamino-2,2-difluor-benzodioxole, Verfahren zu ihrer Herstellung und ihre Verwendung zur Schädlingsbekämpfung

Die vorliegende Erfindung betrifft die neuen 5-Arylamino-2,2-difluor-benzodioxole, Verfahren zu ihrer Herstellung und ihre Verwendung zur Schädlingsbekämpfung.

Es ist bereits bekannt, daß Diarylamine insektizide, akarizide und fungizide Eigenschaften besitzen (vgl. Deutsche Offenlegungsschrift 2 509 416). Ihre Wirkung ist jedoch, vor allem bei niedrigen Aufwandkonzentrationen, nicht voll befriedigend.

Es wurden nun die neuen 5-Arylamino-benzodioxole der allgemeinen Formel I gefunden

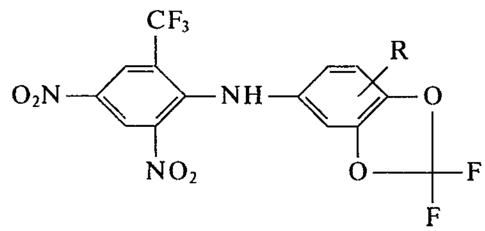

(I)

in welcher

R für Wasserstoff, gegebenenfalls durch Halogen substituiertes Alkyl oder Halogen steht.

Sie weisen ausgezeichnete insektizide, akarizide und fungizide Eigenschaften auf.

Weiterhin wurde gefunden, daß man die neuen Diarylamine der Formel I erhält, wenn man 2-Chlor-3,5-dinitro-benzotrifluorid der Formel II

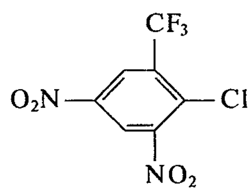

(II)

mit einem 5-Amino-benzodioxol der Formel III,

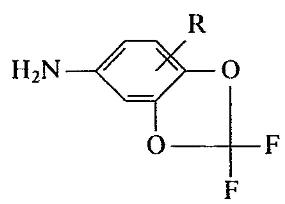

(III)

in welcher

R die oben angegebene Bedeutung hat,

in Gegenwart eines säurebindenden Mittels sowie gegebenenfalls eines Verdünnungsmittels umsetzt.

Überraschenderweise zeigen die neuen Verbindungen der Formel I eine wesentlich höhere insektizide, akarizide und fungizide Potenz als die aus der eingangs erwähnten Offenlegungsschrift bekannten Stoffe, welche die chemisch nächstliegenden Substanzen gleicher Wirkungsrichtung sind. Die erfindungsgemäßen Verbindungen stellen somit eine wertvolle Bereicherung der Technik dar.

Bevorzugt sind Verbindungen der Formel I in welcher R die weiter unten angegebene vorzugsweise Definition besitzt.

Verwendet man 2-Chlor-3,5-dinitro-benzotrifluorid und 5-Amino-2,2-difluorbenzodioxol als Ausgangsstoffe, so läßt sich der Reaktionsablauf durch das folgende Formelschema wiedergeben:

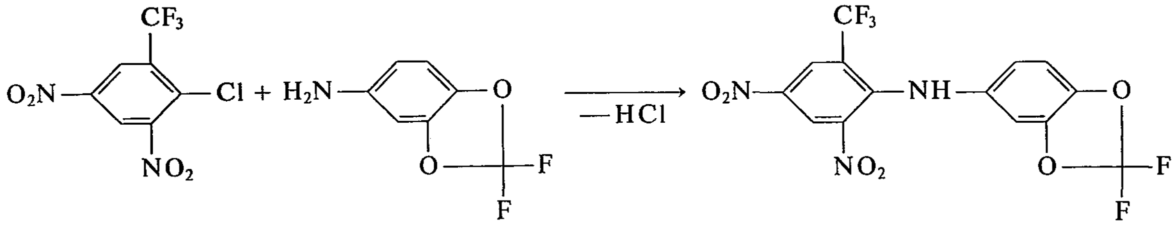


Das als Ausgangsstoff zu verwendende 2-Chlor-3,5-dinitrobenzotrifluorid ist bekannt.

Vorzugsweise werden Verbindungen der Formel III eingesetzt, in denen R für Wasserstoff, Methyl oder Chlor steht. Besonders bevorzugt sind 5-Amino-2,2-difluor-benzodioxol und 5-Amino-6-chlor-2,2-difluor-benzodioxol.

Als Verdünnungsmittel eignen sich inerte organische Lösungsmittel. Bevorzugt werden Dimethylformamid oder Tetrahydrofuran. Zuweilen ist es auch vorteilhaft, in wäßriger Suspension zu arbeiten.

Als säurebindende Mittel eignen sich Basen wie Alkalimetallhydroxide, -carbonate oder -hydride. Bevorzugt verwendet man Natriumhydrogencarbonat, Kaliumhydroxid oder Natriumhydrid.

Die Reaktionstemperatur kann in einem größeren Bereich variiert werden. Im allgemeinen arbeitet man zwischen $-20$ und $+150°$, vorzugsweise zwischen 0 und 100°.

Gewöhnlich werden die Reaktionspartner in äquimolaren Mengen eingesetzt.

Wie bereits erwähnt, sind die Verbindungen der Formel II insektizid, akarizid und fungizid wirksam. Bei höheren Aufwandkonzentrationen sind manche Verbindungen der Formel I auch herbizid wirksam.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe eigenen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den obenerwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophaus, Scutigera spec.

Aus der Ordnung der Symphyla z. B. Scutigerella immaculata.

Aus der Ordnung der Thysanuara z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips fermoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia

brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp. Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkysulfonate, Alkylsulfate, Arylsulfonate sowie Eiweißhydrolysate; als Dispergiermittel kommen in Frage. z. B. Lignin-Sufitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen

Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Bei der Saatgutbehandlung werden im allgemeinen Wirkstoffmengen von 0,001 bis 50 g je Kilogramm Saatgut, vorzugsweise 0,05 bis 5 g, benötigt.

Bei Behandlung des Bodens sind Wirkstoffmengen von 0,00001 bis 0,1 Gew.-%, vorzugsweise von 0,0001 bis 0,02%, am Wirkungsort erforderlich.

## Beispiel A

### Tetranychus-Test (resistent)

| | |
|---|---|
| Lösungsmittel: | 3 Gewichtsteile |
| Emulgator: | 1 Gewichtsteil Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 1, 2.

## Beispiel B

### Myzus-Test

| | |
|---|---|
| Lösungsmittel: | 3 Gewichtsteile |
| Emulgator: | 1 Gewichtsteil Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z. B. die folgenden Verbindungen der Herstellungsbeispiele überlegene Wirksamkeit gegenüber dem Stand der Technik: 1, 2.

## Beispiel C

### Podosphaera-Test (Apfel) / Protektiv

| | |
|---|---|
| Lösungsmittel: | 4,7 Gewichtsteile Aceton |
| Emulgator: | 0,3 Gewichtsteile Alkyl-arylpolyglykoläther |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien

des Apfelmehltauerregers (Podophaera leucotricha) inokuliert und in ein Gewächshaus mit einer Temperatur von 21 – 23° C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen der Herstellungsbeispiele überlegene Wirksamkeit gegenüber dem Stand der Technik: 1,2.

## Beispiel D

### Fusicladium-Test (Apfel) / Protektiv

| | |
|---|---|
| Lösungsmittel: | 4,7 Gewichtsteile Aceton |
| Emulgator: | 0,3 Gewichtsteile Alkyl-arylpolylglykoläther |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 – 20° C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen der Herstellungsbeispiele überlegene Wirksamkeit gegenüber dem Stand der Technik: 1, 2.

## Beispiel E

### Sproßbehandlungs-Test / Getreiderost / protektiv (blattzerstörende Mykose

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20° C und einer 100%igen Luftfeuchtigkeit in ein Gewächshaus.

Nach 10 Tagen Verweilzeit der.Pflanzen bei einer Temperatur von 20° C und einer Luftfeuchtigkeit von 80 – 90% wertet man den Besatz der Pflanzen mit Rostpusteln aus. Der Befallsgrad wird in Prozenten des Befalls der unbehandelten Kontrollpflanzen ausgedrückt. Dabei bedeuten 0% keinen Befall und 100% den gleichen Befallsgrad wie bei der unbehandelten Kontrolle. Der Wirkstoff ist um so wirksamer, je geringer der Rostbefall ist.

Bei diesem Test zeigen z. B. die folgenden Verbindungen der Herstellungsbeispiele überlegen Wirksamkeit gegenüber dem Stand der Technik: 1, 2.

## Beispiel F

### Sproßbehandlungs-Test / Getreidemehltau / protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die

Gerstenpflanzen mit Sporen von Erypsiphe graminis var. hordei.

Nach 6 Tagen Verweilzeit der Pflanzen bei einer Temperatur von 21 – 22° C und einer Luftfeuchtigkeit von 80 – 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen der Herstellungsbeispiele überlegene Wirksamkeit gegenüber dem Stand der Technik: 1, 2.

## Herstellungsbeispiele

1.

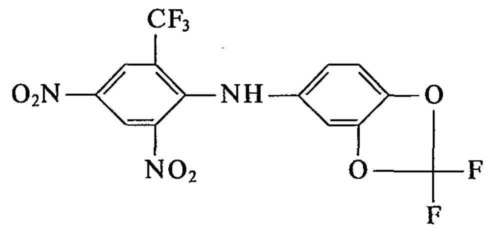

19,0 g (0,07 Mol) 2-Chlor-3,5-dinitro-benzotrifluorid und 12,1 g (0,07 Mol) 5-Amino-2,2-difluor-benzodioxol werden in 100 ml Wasser suspendiert. Man fügt 6 g Natriumhydrogencarbonat zu und erhitzt 2 Stdn. unter Rückfluß. Nach Abkühlen wird abgesaugt und mit Wasser und Methanol gewaschen, wobei man 16,3 g gelbe Kristalle vom Fp. 122° erhält.

2.

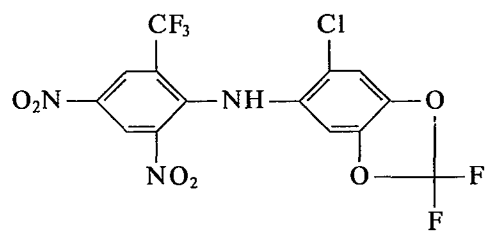

Zu 10,4 g (0,05 Mol) 5-Amino-6-chlor-2,2-difluor-benzodioxol und 5,6 g (0,1 Mol) pulverisiertem Kaliumhydroxid in 100 ml Dimethylformamid tropft man bei 20° unter Rühren eine Lösung von 13,5 g (0,05 Mol) 2-Chlor-3,5-dinitro-benzotrifluorid in 100 ml Dimethylformamid. Man rührt 15 Stdn. bei Raumtemperatur, versetzt mit 50 ml Eisessig und gießt auf Eis, wobei sich das 6-Chlor-2,2-difluor-5-(2,4-dinitro-6-trifluormethylphenylamino)-benzodioxol als dunkles, zähes Öl abscheidet. Molmasse 441, $Cl_1$ (massenspektrometrisch).

**Patentansprüche**

1. 5-Arylamino-benzodioxole der allgemeinen Formel I

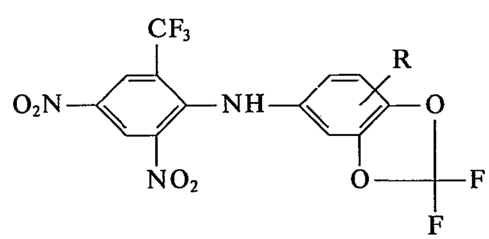

(I)

in welcher

R für Wasserstoff, gegebenenfalls durch Halogen substituiertes Alkyl oder Halogen steht.

2. Verfahren zur Herstellung der 5-Arylamino-benzodioxole der Formel I gemäß Anspruch 1, dadurch gekennzeichnet, daß man 2-Chlor-3,5-dinitro-benzotrifluorid der Formel II

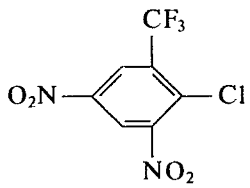

(II)

mit einem 5-Amino-benzodioxol der Formel III

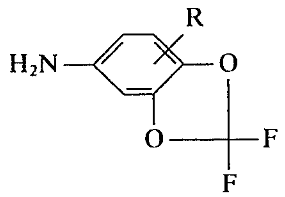

(III)

in welcher

R die oben angegebene Bedeutung hat,

in Gegenwart eines säurebindenden Mittels sowie gegebenenfalls eines Verdünnungsmittels umsetzt.

3. Schädlingsbekämpfungsmittel, gekennzeichnet durch einen Gehalt an mindestens einem 5-Arylamino-benzodioxol der Formel I gemäß Anspruch 1.

4. Verwendung von 5-Arylamino-benzodioxolen der Formel I gemäß Anspruch 1 zur Bekämpfung von Schädlingen.

5. Verfahren zur Herstellung von Schädlingsbekämpfungsmitteln, dadurch gekennzeichnet, daß man 5-Arylamino-benzodioxole der Formel I gemäß Anspruch 1 mit Streckmitteln und/oder oberflächenaktiven Mitteln vermischt.

**Claims**

1. 5-Arylamino-benzodioxoles of the general formula I

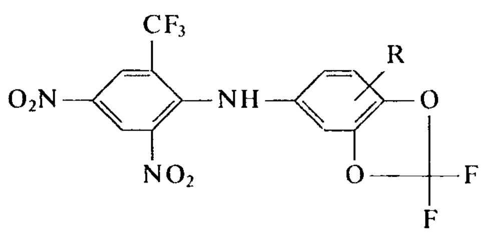

(I)

in which

R represents hydrogen, alkyl which is optionally substituted by halogen or halogen.

2. Process for the preparation of the 5-arylamino-benzodioxoles of the formula I according to Claim 1, characterised in that 2-chloro-3,5-dinitro-benzotrifluoride of the formula II

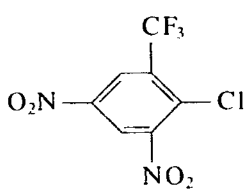

(II)

is reacted with a 5-amino-benzodioxole of the formula III

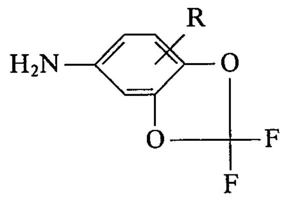

(III)

in which

R has the meaning indicated above,

in the presence of an acid-binding agent and, if appropriate, in the presence of a diluent.

3. Pesticides characterised in that they contain at least one 5-arylamino-benzodioxole of the formula I according to Claim 1.

4. Use of 5-arylamino-benzodioxoles of the formula I according to Claim 1 for combating pests.

5. Process for the preparation of pesticides, characterised in that 5-arylamino-benzodioxoles of the formula I according to Claim 1 are mixed with extenders and/or surface-active agents.

**Revendications**

1. 5-arylaminobenzodioxoles caractérisés en ce qu'ils répondent à la formule générale

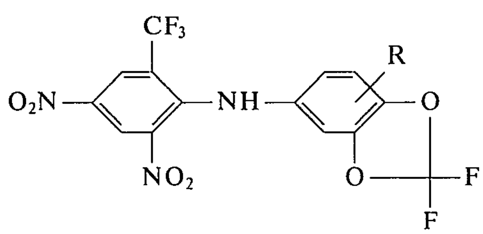

(I)

dans laquelle

R représente l'hydrogène, un reste alkyle éventuellement substitué par un halogène ou un halogène.

2. Procédé pour la préparation des 5-arylaminobenzodioxoles de formule I selon la revendication 1, caractérisé en ce que l'on fait réagir le fluorure de 2-chloro-3,5-dinitrobenzényle de formule II

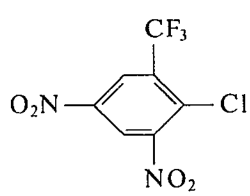

(II)

avec un 5-aminobenzodioxole de formule III

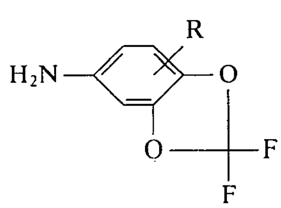

(III)

dans laquelle

R a la signification indiquée ci-dessus, en présence d'un accepteur d'acide, ainsi éventuellement que d'un agent diluant.

3. Agents de lutte contre les parasites, caractérisés en ce qu'ils contiennent au moins un

5-arylaminobenzodioxole de formule I selon la revendication 1.

4. Application des 5-arylaminobenzodioxoles de formule I selon la revendication I pour la lutte contre les parasites.

5. Procédé pour la préparation d'agents de lutte contre les parasites, caractérisé en ce que l'on mélange des 5-arylaminobenzodioxoles de formule I selon la revendication 1 avec des agents diluants et/ou des agents tensioactifs.